# FASCICULE DE BREVET EUROPEEN

(11) **EP 2 978 525 B1**
(45) Date de publication et mention de la délivrance du brevet: **13.06.2018**
(21) Numéro de dépôt: 14713142.9
(22) Date de dépôt: 28.03.2014
(51) Int. Cl.: B01J 35/00, B01J 23/745, B01J 23/755, B01J 27/051, B01J 27/057, B01J 35/10, B01J 23/28, B01J 37/02, B01J 27/18, C07C 319/06, C07C 319/02

(54) **CATALYSEUR POUR LA SYNTHÈSE DE MÉTHYL MERCAPTAN ET PROCÉDÉ DE PRODUCTION DE MÉTHYL MERCAPTAN À PARTIR DE GAZ DE SYNTHÈSE ET DE SULFURE D' HYDROGÈNE**
KATALYSATOR ZUR SYNTHESE VON METHYLMERCAPTAN UND VERFAHREN ZUR HERSTELLUNG VON METHYLMERCAPTAN AUS SYNTHESEGAS UND SCHWEFELWASSERSTOFF
CATALYST FOR THE SYNTHESIS OF METHYL MERCAPTAN AND PROCESS FOR PRODUCING METHYL MERCAPTAN FROM SYNTHESIS GAS AND HYDROGEN SULPHIDE

(30) Priorité: 29.03.2013 FR 1352871
(43) Date de publication de la demande: 03.02.2016
(73) Titulaire: Arkema France, 92700 Colombes (FR)
(72) Inventeur: FREMY, Georges, F-64390 Sauveterre de Bearn (FR); BARRE, Patrice, F-64140 Lons (FR); SANCHOU, Karine, F-64000 Pau (FR); CORDOVA, Alexia, F-59800 Lille (FR); LAMONIER, Carole, F-59280 Armentières (FR); BLANCHARD, Pascal, F-62300 Lens (FR)
(74) Mandataire: Casalonga
(86) Numéro de dépôt international: PCT/EP2014/056343
(87) Numéro de publication internationale: WO 2014/154885

(56) Documents cités:
- EP-A1- 0 571 090
- US-A1- 2007 213 564
- YANG Y-Q ET AL: "THE CATALYTIC PROPERTIES OF SUPPORTED K2MOS4/SIO2 CATALYST FOR METHANETHIOL SYNTHESIS FROM HIGH H2S-CONTENT SYNGAS", CATALYSIS LETTERS, SPRINGER NEW YORK LLC, UNITED STATES, vol. 54, no. 1/02, 1 septembre 1998 (1998-09-01), pages 65-68, XP000782209, ISSN: 1011-372X, DOI: 10.1023/A:1019071704226
- Y-Q YANG ET AL: "Study of the supported K2MoO4 catalyst for methanethiol synthesis by one step from high H2S-containing syngas", CATALYSIS LETTERS, SPRINGER NEW YORK LLC, UNITED STATES, vol. 74, no. 3-4, 1 janvier 2001 (2001-01-01), pages 221-225, XP002322024, ISSN: 1011-372X, DOI: 10.1023/A:1016614004566
- CAROLE LAMONIER ET AL: "Specific tuning of acid/base sites in apatite materials to enhance their methanol thiolation catalytic performances", CATALYSIS TODAY, vol. 164, no. 1, 4 novembre 2010 (2010-11-04), pages 124-130, XP028384295, ISSN: 0920-5861, DOI: 10.1016/J.CATTOD.2010.10.035 [extrait le 2010-10-15]
- OLIVER Y GUTIRREZ ET AL: "Synthesis of methyl mercaptan from carbonyl sulfide over sulfide KMoO/SiO", JOURNAL OF CATALYSIS, ACADEMIC PRESS, DULUTH, MN, US, vol. 280, no. 2, 26 mars 2011 (2011-03-26) , pages 264-273, XP028091574, ISSN: 0021-9517, DOI: 10.1016/J.JCAT.2011.03.027 [extrait le 2011-04-02]

## Description

Le travail qui a conduit à cette invention a reçu un financement de la part de l'Union Européenne dans le cadre du 7ième Programme Cadre (FP7/2007-2013) sous le numéro de projet N°241718 EUROBIOREF.

La présente invention porte sur un catalyseur spécifique à base de molybdène et de potassium utile pour la production de méthyl mercaptan à partir de gaz de synthèse et de sulfure d'hydrogène, et sur son procédé de préparation.

L'invention porte également sur un procédé de production de méthyl mercaptan mettant en oeuvre ce catalyseur.

Le méthyl mercaptan présente un grand intérêt industriel, en particulier comme matière première de la synthèse de méthionine, acide aminé essentiel fortement utilisé dans l'alimentation animale. Le méthyl mercaptan est également une matière première pour de nombreuses autres molécules, notamment le diméthyldisulfure (DMDS), additif de sulfuration de catalyseur d'hydrotraitement de coupes pétrolières, entre autres applications.

Le méthyl mercaptan est couramment produit en gros tonnage industriellement à partir de méthanol et de sulfure d'hydrogène. Il peut se révéler intéressant économiquement de vouloir produire le méthyl mercaptan directement à partir de monoxyde de carbone, d'hydrogène et de sulfure d'hydrogène selon le schéma de synthèse suivant :

CO+2H₂+H₂S→CH₃SH+H₂O (1)

Le sous-produit principal de cette synthèse est le dioxyde de carbone. En effet, l'oxysulfure de carbone (COS) est considéré comme l'intermédiaire réactionnel qui mène au méthyl mercaptan après hydrogénation selon les schémas suivants :

CO+H₂S → COS+H₂ (2)

COS+3H₂ → CH₃SH+H₂O (3)

Le dioxyde de carbone est quant à lui issu de deux réactions parasites :

CO+H₂O → CO₂+H₂ (4)

et

COS+H₂O → CO₂+H₂S (5)

Ces deux réactions parasites, qui consomment la matière première principale : le monoxyde de carbone et l'intermédiaire réactionnel : l'oxysulfure de carbone sont dues à la présence inéluctable d'eau, coproduite lors de la synthèse du méthyl mercaptan. Le dioxyde de carbone peut éventuellement être recyclé pour produire également du méthyl mercaptan selon le schéma suivant :

CO₂+3H₂+H₂S → CH₃SH+2H₂O (6)

Mais cette réaction est connue pour être plus lente que celle à partir du monoxyde de carbone. Il y a donc tout intérêt à ce que la production de dioxyde de carbone soit la plus faible possible à la sortie du réacteur de synthèse du méthyl mercaptan.

Il est connu du document WO2005/040082 plusieurs catalyseurs pour la synthèse de méthyl mercaptan à partir de gaz de synthèse et de sulfure d'hydrogène.

Ce document divulgue notamment l'utilisation de catalyseur comprenant un composant actif à base de Mo-O-K, un promoteur actif et éventuellement un support. Les catalyseurs exemplifiés sont de nature chimique différente, tels que K₂MoO₄/Fe₂O₃/NiO ou encore K₂MoO₄/CoO/CeO₂/SiO₂ chacun supporté sur de la silice. Ce dernier conduit à un ratio de sélectivité CO₂/MeSH de 0,88 à 333°C.

Il est également connu du document US2010/0286448 une famille de catalyseurs composée d'un support poreux sur lequel a été déposé électrolytiquement un métal. Le K₂MoO₄ a ensuite été imprégné sur ce support ainsi qu'un autre oxyde métallique en tant que promoteur. L'exemple 15 de ce document décrit la préparation de K₂MoO₄/NiO/CoSiO₂. Le ratio de sélectivité CO₂/MeSH avec ce catalyseur complexe, est de 0,65.

Le document US 2010/0094059 décrit des catalyseurs à base de K₂MoO₄ supporté, le support poreux utilisé seul ou en mélange étant choisi parmi du SiO₂, Al₂O₃, TiO₂, Al₂O₃/SiO₂, ZrO₂, des zéolites, des matériaux carbonés. De l'oxyde de tellure (TeO₂) est utilisé en tant que promoteur. Les ratios de sélectivité CO₂/MeSH sont compris entre 0,60 et 0,77 mesurés à 300°C.

Par ailleurs, dans les articles scientifiques « The catalytic properties of supported K2MoS4/SiO2 catalyst for methanethiol synthesis from high H2S-content syngas » (Catalysis Letters Vol.54, 1998, 65-68) et « Study of the supported K2MoO4 catalyst for methanethiol synthesis by one step from high H2S-containing syngas » (Catalysis Letters Vol.74, No. 3-4, 2001, 221-225), Yang et al. divulguent respectivement les propriétés catalytiques de catalyseurs à base de K₂MoS₄ et de K₂MoO₄ supportés sur de la silice dans la synthèse du méthyl mercaptan.

Il est également connu du document EP0571090 d'utiliser des catalyseurs supportés par du phosphate dans la conversion des alcools aliphatiques inférieurs en hydrocarbures. Enfin, l'article scientifique, « Specific tuning of acid/base sites in apatite materials to enhance their methanol thiolation catalytic performances » (Catalysis Today, Vol,164, 2011, 124-130) enseigne l'utilisation de catalyseurs à base d'apatite pour la synthèse du méthanethiol. Il a été observé à partir de l'enseignement de ces documents que l'association de catalyseurs de structure spécifique, de promoteurs et de supports, chacun d'entre eux étant minutieusement sélectionné permet d'atteindre des ratios de sélectivité intéressants.

Or, il existe un réel besoin de catalyseur simple à synthétiser et conduisant à de très bonnes sélectivités. Ce problème technique a été résolu par un catalyseur à base de molybdène et de potassium supporté par de l'hydroxyapatite.

Il a été observé que le catalyseur selon l'invention est plus facile à préparer, étant donné que la présence d'un promoteur n'est pas indispensable. Il est moins coûteux que ceux divulgués dans les documents précités. Et enfin, il conduit à de très bonnes sélectivités CO₂/MeSH.

L'invention porte également sur le procédé de préparation de ce catalyseur.

L'invention a aussi pour objet un procédé de production de méthyl mercaptan à partir de gaz de synthèse et du sulfure d'hydrogène mettant en oeuvre le catalyseur selon l'invention.

L'invention porte aussi sur l'utilisation du catalyseur tel que défini ci-dessus pour la synthèse de méthyl mercaptan à partir de gaz de synthèse et du sulfure d'hydrogène.

L'invention porte enfin sur l'utilisation d'hydroxyapatite en tant que support pour la préparation de catalyseur pour la production de méthyl mercaptan, et notamment dans un procédé catalytique par réaction d'oxyde de carbone, de soufre et/ou de sulfure d'hydrogène et d'hydrogène.

D'autres caractéristiques, aspects, objets et avantages de la présente invention apparaîtront encore plus clairement à la lecture de la description et des exemples qui suivent.

D'autre part, tout intervalle de valeurs désigné par l'expression "entre a et b" représente le domaine de valeurs allant de plus de a à moins de b (c'est-à-dire bornes a et b exclues), tandis que tout intervalle de valeurs désigné par l'expression "de a à b" signifie le domaine de valeurs allant de a jusqu'à b (c'est-à-dire incluant les bornes strictes a et b).

### Catalyseur

La présente invention a pour objet un catalyseur.

Ce catalyseur comprend un composant actif à base de molybdène et de potassium, et un support à base d'hydroxyapatite.

### Composant actif

Le composant actif présent dans le catalyseur selon l'invention comprend du molybdène et du potassium au sein d'un même composant.

De préférence, le composant actif à base de molybdène et de potassium est choisi parmi des composés à base de Mo-S-K, des composés à base de Mo-O-K et leurs mélanges.

Le composant actif à base de Mo-S-K peut être obtenu par dépôt et calcination de précurseurs K₂MoS₄ ou (NH₄)₂ MoS₄ additionnés de K₂CO₃ imprégné séparément sur le support.

Le composant actif à base de Mo-O-K peut être obtenu par dépôt et calcination de précurseurs K₂MoO₄ ou (NH₄)₂ MoO₄ additionnés de K₂CO₃ imprégné séparément sur le support.

Il est également possible d'utiliser en tant que réactif de l'heptamolybdate d'ammonium (NH₄)₆Mo₇O₂₄.4H₂O, en présence d'un sel de potassium comme par exemple le nitrate de potassium KNO₃, le carbonate de potassium K₂CO₃ ou encore la potasse KOH.

Ces composés sont des précurseurs des phases actives à base de Mo-S-K et Mo-O-K respectivement. Les phases actives sont obtenues après prétraitement *in situ* des précurseurs, avec par exemple une procédure consistant dans une première étape en un séchage à l'azote à 250°C, suivie par une sulfuration avec du sulfure d'hydrogène à la même température pendant 1 heure, puis une étape de réduction/sulfuration avec H₂/H₂S à 350°C pendant 1 heure.

### Support

Le support du catalyseur selon l'invention est de l'hydroxyapatite de formule Ca₁₀(PO₄)₆(OH)₂, avantageusement une hydroxyapatite stoechiométrique.

De préférence, l'hydroxyapatite utile selon la présente invention présente un rapport molaire Ca/P allant de 1,5 à 2,1, et plus particulièrement égal à 1,67, correspondant à la valeur attendue pour une hydroxyapatite stoechiométrique.

De préférence, le rapport pondéral du catalyseur selon l'invention est :

K₂MoS₄/Ca₁₀(PO₄)₆(OH)₂=31,3/100

K₂MoO₄/Ca₁₀(PO₄)₆(OH)₂= 50,7/100

L'activité catalytique peut être améliorée en utilisant un matériau support présentant une aire spécifique supérieure à 25 m²/g.

De préférence, les supports d'hydroxyapatite selon l'invention présentent une aire spécifique d'au moins 40 m²/g, plus particulièrement l'aire spécifique va de 40 m²/g à 300 m²/g et un rapport molaire Ca/P de 1,67.

La structure du support peut être une structure tridimensionnelle, de forme sphérique, cylindrique, en forme d'anneau, d'étoile, de granulats ou de toute autre forme tridimensionnelle, ou bien sous forme d'une poudre, qui peut être pressée, extrudée, granulée, dans une forme tridimensionnelle.

De préférence, les particules de catalyseur ont une distribution en taille de particule uniforme en diamètre de 0,1 mm à 20,0 mm mesurée par granulométrie sur tamis.

### Promoteur

De préférence, le catalyseur selon l'invention consiste en un composant actif à base de molybdène et de potassium et un support à base d'hydroxyapatite.

Toutefois, il est possible d'envisager la présence d'un promoteur, tel que l'oxyde de tellure, l'oxyde de nickel ou l'oxyde de fer connu de l'homme du métier.

### Procédé de préparation du catalyseur

L'invention porte également sur le procédé de préparation du catalyseur selon l'invention. Ce procédé comprend les étapes successives suivantes :
- préparation du précurseur de la phase active,
- préparation du support, et
- imprégnation à sec du support par le précurseur de la phase active.

### Préparation du précurseur de la phase active

### 1/Mo-O-K

1. Le sel de K₂MoO₄ est un sel commercial. Pour la préparation du catalyseur à base de Mo-O-K, une quantité fixée de K₂MoO₄ est dissoute dans un volume d'eau pour obtenir une solution de concentration souhaitée, tel que par exemple une concentration allant de 0,5 à 1,0 g/mL.
2. Il est également possible de partir des sels de molybdène et de potassium séparés, c'est-à-dire ne faisant pas partie du même composé. Pour cette synthèse, une solution à base de molybdène est préparée par addition d'heptamolybdate d'ammonium dans l'eau afin d'obtenir une concentration en MoO₃ allant de 22 à 33% en poids.

Parallèlement, une solution à base de potassium a été préparée par addition de nitrate de potassium dans l'eau, afin d'obtenir une concentration en K₂O allant de 31 à 43 % en poids.

### 2/Mo-S-K

La synthèse de K₂MoS₄ se fait généralement en deux étapes.

La première étape implique la préparation de tétrathiomolybdate d'ammonium (ATTM), et la deuxième étape est la synthèse de tétrathiomolybdate de potassium (K₂MoS4) à partir du sel préparé dans la première étape.

Pour la préparation d'ATTM, du sulfure d'hydrogène est laissé buller en continu dans une solution aqueuse ammoniacale à 25%, dans laquelle a été dissous de l'heptamolybdate d'ammonium (HMA). La température de la solution augmente, indiquant l'exothermicité de la réaction. Le bullage du sulfure d'hydrogène est stoppé, lorsque la température diminue (généralement au bout d'une heure).

La solution contient alors des cristaux rouges avec des reflets verts, qui correspondent au tétrathiomolybdate d'ammonium.

La deuxième étape consiste à un échange ionique entre les ions ammonium du sel de tétrathiomolybdate d'ammonium obtenu et des ions potassium, qui proviennent d'une solution d'hydroxyde de potassium. Les sels obtenus sont alors stockés sous vide. Une quantité de tétrathiomolybdate de potassium est dissoute dans l'eau.

Le sel de potassium utile dans le catalyseur selon la présente invention peut provenir des composés suivants : l'acétate de potassium (KAc), l'oxalate de potassium (K₂C₂O₄), l'hydroxyde de potassium (KOH), le carbonate de potassium (K₂CO₃), le nitrate de potassium (KNO₃), et le bicarbonate de potassium (KHCO₃).

### Préparation du support

La préparation de l'hydroxyapatite, qui constitue le support du catalyseur est effectuée par une méthode de coprécipitation. Une solution aqueuse de nitrate de calcium Ca(NO₃)₂ a été ajoutée goutte à goutte dans une solution d'hydrogénophosphate d'ammonium (NH₄)H₂PO₄ en agitant. La température est maintenue à 100°C et le pH est maintenu à 10 avec addition d'une solution d'ammoniaque (25%).

Le précipité blanc résultant est filtré, lavé, séché à 80°C pendant une nuit et calciné à 400°C. L'hydroxyapatite Ca₁₀(PO₄)₆(OH)₂ a été obtenue avec un ratio molaire Ca/P égal à 1,67 correspondant à la valeur attendue pour une hydroxyapatite stoechiométrique.

### Imprégnation à sec du support par le précurseur de la phase active

### 1/Mo-O-K

La méthode d'imprégnation à sec est utilisée pour la préparation du catalyseur. La solution de K₂MoO₄ est imprégnée en une étape sur le support. Lorsque les solutions contenant le potassium et le molybdène sont distinctes, l'imprégnation s'effectue en 2 étapes.

### 2/Mo-S-K

Une solution de tétrathiomolybdate de potassium est ensuite imprégnée sur l'hydroxyapatite. La teneur en molybdate dans le catalyseur dépend de la solubilité du K₂MoS₄ ou de K₂MoO₄ et du volume poreux du support.

La solubilité de K₂MoS₄ est entre 0,25 g/mL et 0,50 g/mL (0,35 g/mL) et la solubilité de K₂MoO₄ est entre 0,50 g/mL et 1,50 g/mL (0,90 g/mL). Le volume poreux du support est entre 0,8 mL/g et 2,2 mL/g.

Par conséquent, le volume de solution utilisé est calculé de manière à obtenir le rapport pondéral souhaité, et de préférence le rapport pondéral tel que défini ci-dessus.

Après imprégnation, le solide subit une étape de maturation pendant 2 heures, puis un séchage en étuve à 80 °C pendant 24 heures et une calcination sous flux de gaz (typiquement l'air) à 490 °C pendant 4 heures. Si une deuxième étape d'imprégnation est nécessaire, le solide subit à nouveau les étapes de maturation, séchage et calcination.

### Procédé de production de méthylmercaptan

L'invention porte sur un procédé de production de méthyl mercaptan dans un procédé catalytique par réaction d'oxyde de carbone, de soufre et/ou de sulfure d'hydrogène et d'hydrogène, comprenant l'utilisation d'un catalyseur tel que défini ci-dessus.

Les rapports molaires de CO ou CO₂/H₂S/H₂ vont de 1/1/0 à 1/8/8, ou lorsque du soufre est utilisé en remplacement du sulfure d'hydrogène, les rapports molaires des réactifs CO ou CO₂/H₂S/H₂/S vont de 1/1/0/1 à 1/8/8/8.

De préférence, les rapports molaires CO ou CO₂/H₂S/H₂ vont de 1/2/1 à 1/4/4, lorsque du soufre est utilisé en remplacement du sulfure d'hydrogène, les rapports molaires des réactifs CO ou CO₂/H₂S/H₂/S de 1/2/2/1 a 1/4/4/4.

Ces rapports molaires tiennent compte du CO₂. De ce fait, ils considèrent à la fois le schéma de synthèse (1) et le schéma de synthèse (6).

De préférence, la réaction peut se dérouler dans des réacteurs à lit fixe tubulaire, multitubulaire, à micro-canaux à paroi catalytique ou à lit fluidisé.

L'invention a également pour objet l'utilisation du catalyseur tel que défini ci-dessus pour la production de méthyl mercaptan à partir de gaz de synthèse et de sulfure d'hydrogène.

La présente invention va être maintenant décrite dans les exemples ci-dessous, de tels exemples étant donnés à but uniquement illustratif, et bien évidemment non limitatif.

### EXEMPLES

### Exemple 1

Le catalyseur selon l'invention est préparé selon la méthode d'imprégnation à sec, telle que définie ci-dessus.

Le catalyseur ainsi obtenu présente les caractéristiques suivantes :

**Tableau 1 : Analyse élémentaire du catalyseur**

| Catalyseur (% en poids) | Composition chimique | | | |
|---|---|---|---|---|
| | Mo | K | s | N |
| K₂MoS₄/Hap | 9,9 | 8,1 | 13,3 | <0,10 |

### Exemple 2 : Le catalyseur utilisé est du K₂MoO₄ sur de l'hydroxyapatite

### Exemple 3 : Le catalyseur testé est du K₂MoO₄ sur SiO₂

### Exemple 4 : Le catalyseur testé est du K₂MoS₄ sur Al₂O₃

### Exemple 5 : Le catalyseur testé est du K₂MoO₄ sur Al₂O₃.

### Evaluation des catalyseurs

Les catalyseurs sont évalués dans une réaction de production de méthyl mercaptan dans un réacteur à lit fixe dans les conditions suivantes :
Température : 280°C,
Pression : 10 bars,
Composition du gaz d'alimentation CO/H₂/H₂S=1/2/1 (v par v),
GHSV (Gas Hourly Space Velocity)= 1333h⁻¹

Les réactifs et les produits ont été analysés en ligne par chromatographie gazeuse.

Avant le test, les catalyseurs ont été activés *in situ* avec une procédure consistant dans une première étape en un séchage à l'azote à 250°C, suivie par une sulfuration avec du sulfure d'hydrogène à la même température pendant 1 heure, puis une étape de réduction/sulfuration avec H₂/H₂S à 350°C pendant 1 heure.

Les résultats figurent dans le tableau 2 ci-dessous.

**TABLEAU 2 : Résultats des tests catalytiques**

| Exemples | Catalyseur | Sélectivités molaires (%) | | | Ratio CO₂/CH₃SH |
|---|---|---|---|---|---|
| | | CH₃SH | cos | CO₂ | |
| 1 (inv) | K₂MoS₄/Hap | 44,1 | 23,3 | 32,6 | 0,74 |
| 2 (inv) | K₂MoO₄/Hap | 43,3 | 23,6 | 31,9 | 0,74 |
| 3 (comp) | K₂MoO₄/SiO₂ | 48,8 | 5,3 | 45,3 | 0,93 |
| 4 (comp) | K₂MoS₄/Al₂O₃ | 45,0 | 7,3 | 46,6 | 1,04 |
| 5 (comp) | K₂MoO₄/Al₂O₃ | 47,0 | 3,4 | 49,6 | 1,06 |

Les résultats présentés dans le tableau 2 montrent que les catalyseurs selon l'invention (exemples 1 et 2) donnent des sélectivités en CO₂ (produit indésirable) nettement inférieures comparativement aux catalyseurs sur des supports de l'art antérieur (silice : exemple 3 ou alumine : exemples 4 et 5).

Les sélectivités sont comparées à isoconversion de monoxyde de carbone, cette conversion étant exprimée par m² d'aire spécifique du catalyseur.

En comparant les résultats obtenus avec les catalyseurs 1 et 4, on observe une amélioration de 30% en terme de ratio, cette amélioration étant liée au choix de l'hydroxyapatite en tant que support.

Le même constat est fait en comparant l'exemple 2 selon l'invention et les exemples 3 et 5.

On constate ainsi une sélectivité accrue en méthyl mercaptan par rapport au dioxyde de carbone produit selon une réaction secondaire.

Il est à noter que cette sélectivité est obtenue sans aide de promoteur tel que l'oxyde de tellure, l'oxyde de nickel ou l'oxyde de fer tel que décrit dans l'art antérieur.

## Revendications

1. Catalyseur comprenant un composant actif à base de molybdène et de potassium et un support à base d'hydroxyapatite.

2. Catalyseur selon la revendication 1, caractérisé en ce le support du catalyseur est de l'hydroxyapatite de formule Ca₁₀(PO₄)₆(OH)₂ stoechiométrique.

3. Catalyseur selon la revendication 1 ou 2, **caractérisé en ce que** le composant actif à base de molybdène et de potassium est choisi parmi des composés à base de Mo-S-K, des composés à base de Mo-O-K et leurs mélanges.

4. Catalyseur selon la revendication 3, **caractérisé en ce que** le précurseur du composant actif à base de Mo-S-K est de structure K₂MoS₄.

5. Catalyseur selon la revendication 4, caractérisé en ce le rapport pondéral du catalyseur selon l'invention est
K₂MoS₄/Ca₁₀(PO₄)₆(OH)2= 31,3/100

6. Catalyseur selon la revendication 3, **caractérisé en ce que** le précurseur du composant actif à base de Mo-O-K est de structure K₂MoO₄.

7. Catalyseur selon la revendication 6, caractérisé en ce le rapport pondéral du catalyseur selon l'invention est :
K₂MoO₄/Ca₁₀(PO₄)₆(OH)₂= 50,7/100

8. Procédé de préparation du catalyseur tel que défini à l'une quelconque des revendications 4 à 7, **caractérisé en ce qu'**il comprend les étapes suivantes :
- préparation du précurseur de la phase active
- préparation du support, et
- imprégnation à sec du support par le précurseur de la phase active.

9. Procédé de production de méthyl mercaptan dans un procédé catalytique par réaction d'oxyde de carbone, de soufre et/ou de sulfure d'hydrogène et d'hydrogène, comprenant l'utilisation d'un catalyseur tel que défini à l'une quelconque des revendications 1 à 7.

## Patentansprüche

1. Katalysator, umfassend eine aktive Komponente auf Basis von Molybdän und Kalium und einen Träger auf Basis von Hydroxyapatit.

2. Katalysator nach Anspruch 1, **dadurch gekennzeichnet, dass** es sich bei dem Katalysatorträger um Hydroxyapatit mit der stöchiometrischen Formel Ca₁₀(PO₄)₆(OH)₂ handelt.

3. Katalysator nach Anspruch 1 oder 2, **dadurch gekennzeichnet, dass** die aktive Komponente auf Basis von Molybdän und Kalium aus Verbindungen auf Basis von Mo-S-K, Verbindungen auf Basis von Mo-O-K und Mischungen davon ausgewählt ist.

4. Katalysator nach Anspruch 3, **dadurch gekennzeichnet, dass** die Vorstufe der aktiven Komponente auf Basis von Mo-S-K die Struktur K₂MoS₄ aufweist.

5. Katalysator nach Anspruch 4, **dadurch gekennzeichnet, dass** das Gewichtsverhältnis des erfindungsgemäßen Katalysators
K₂MoS₄/Ca₁₀(PO₄)₆(OH)₂ = 31,3/100
beträgt.

6. Katalysator nach Anspruch 3, **dadurch gekennzeichnet, dass** die Vorstufe der aktiven Komponente auf Basis von Mo-O-K die Struktur K₂MoO₄ aufweist.

7. Katalysator nach Anspruch 6, **dadurch gekennzeichnet, dass** das Gewichtsverhältnis des erfindungsgemäßen Katalysators
K₂MoO_{4/}Ca₁₀(PO₄)₆(OH)₂ = 50,7/100
beträgt.

8. Verfahren zur Herstellung des Katalysators gemäß einem der Ansprüche 4 bis 7, **dadurch gekennzeichnet, dass** es die folgenden Schritte umfasst:
- Herstellung der Vorstufe der aktiven Phase,
- Herstellung des Trägers und
- Trockenimprägnierung des Trägers mit der Vorstufe der aktiven Phase.

9. Verfahren zur Herstellung von Methylmercaptan in einem katalytischen Verfahren durch Umsetzung von Kohlenstoffoxid, Schwefel und/oder Schwefelwasserstoff und Wasserstoff, bei dem man einen Katalysator gemäß einem der Ansprüche 1 bis 7 verwendet.

## Claims

1. Catalyst comprising a molybdenum- and potassium-based active component and a hydroxyapatite-based support.

2. Catalyst according to Claim 1, **characterized in that** the catalyst support is hydroxyapatite having stoichiometric formula Ca₁₀(PO₄)₆(OH)₂.

3. Catalyst according to Claim 1 or 2, **characterized in that** the molybdenum- and potassium-based active component is chosen from compounds based on Mo-S-K, compounds based on Mo-O-K, and their mixtures.

4. Catalyst according to Claim 3, **characterized in that** the precursor for the Mo-S-K based active component has structure K₂MoS₄.

5. Catalyst according to Claim 4, **characterized in that** the weight ratio for the catalyst according to the invention is
K₂MoS₄/Ca₁₀(PO₄)₆(OH)₂= 31.3/100

6. Catalyst according to Claim 3, **characterized in that** the precursor for the Mo-O-K based active component has structure K₂MoO₄.

7. Catalyst according to Claim 6, **characterized in that** the weight ratio for the catalyst according to the invention is:
K₂MoO₄/Ca₁₀(PO₄)₆(OH)₂= 50.7/100

8. Preparation process for the catalyst as defined in any of Claims 4 to 7, **characterized in that** it comprises the following steps:
- preparing the precursor for the active phase
- preparing the support, and
- dry impregnating the support with the active phase precursor.

9. Production process for methyl mercaptan in a catalytic process by reacting carbon oxide, sulfur and/or hydrogen sulfide and hydrogen, comprising the use of a catalyst as defined in any of Claims 1 to 7.
